## Europäisches Patentamt

## European Patent Office

## Office européen des brevets

(19)

(11) Publication number: **0 226 354**

**A2**

(12) # EUROPEAN PATENT APPLICATION

(21) Application number: **86309182.3**

(22) Date of filing: **25.11.86**

(51) Int. Cl.⁴: **C 12 P 1/00**
**//C12P17/18, C12N5/00,**
**(C12P1/00, C12R1:91)**

(30) Priority: **05.12.85 CA 496984**

(43) Date of publication of application:
**24.06.87 Bulletin 87/26**

(84) Designated Contracting States:
**AT CH DE FR GB IT LI NL SE**

(71) Applicant: **Canadian Patents and Development Limited**
**Société Canadienne des Brevets et d'Exploitation Limitée**
**275 Slater Street**
**Ottawa Ontario, K1A 0R3(CA)**

(72) Inventor: **Constabel, Friedrich**
**2004-14th Street**
**Saskatoon Sadkatchewan, S7H 0B2(CA)**

(72) Inventor: **Kurz, Wolfgang G.W.**
**514 Copland Crescent**
**Saskatoon Sadkatchewan, S7H 2Z5(CA)**

(72) Inventor: **Ellert, Udo F.K.**
**Inst. für Pharmazeutische Biol. Mendelssohnsstr. 1**
**D-3300 Braunschweig(DE)**

(74) Representative: **Lambert, Hugh Richmond et al,**
**D. YOUNG & CO. 10 Staple Inn**
**London, WC1V 7RD(GB)**

(54) **Method for the semi-continuous production of phytochemicals by plant cells cultured in vitro.**

(57) Phytochemical products are obtained by a semi-continuous method of culturing plant cells in vitro by introducing into the medium an elicitor capable of stimulating the production of the phytochemical, separating intact cells and product phytochemical from the medium, replenishing the medium at least once and optionally adding additional elicitor, and recovering excess cells from the replenished medium and further product phytochemical.

## NOVEL SEMICONTINUOUS PROCESS

FIG. IB

# METHOD FOR THE SEMI-CONTINUOUS PRODUCTION OF PHYTOCHEMICALS
## BY PLANT CELLS CULTURED IN VITRO

0226354

This invention concerns methods for the semi-continuous production of phytochemicals by plant cells cultured in vitro.

To date the general practice has been to produce such phytochemicals by extraction from cells in stationary growth phase. Commonly, production is only achieved after cells have been transferred from a growth medium to a production medium. After metabolite accumulation in the cells, these are harvested for extraction of the desired product. Subsequently, a new batch of cells has to be grown from an initial inoculum and such a process is therefore discontinuous, commonly requiring 4-5 weeks for culture development and metabolite accumulation. Problems, therefore, with these general practices include (a) the need to transfer cells from a growth medium to a production medium, (b) length of time required (usually up to 14 days) to induce the production of phytochemicals and (c) the need to extract the phytochemicals from the cells by means of sacrificing the cells and to grow the culture anew.

This invention concerns the elimination of the need to transfer cells from growth to production medium, the accelerated production of phytochemicals and further concerns the extraction of phytochemicals from the medium and harvesting excess cells.

More particularly, the invention is based on the surprising discovery that in vitro cell cultures can be subjected to successive periods of elicitation, i.e. treatment with an elicitor which is a biological inducer/catalyst which induces or triggers defence mechanisms in plants, including the production of phytochemicals, many of which are defence chemicals. In support of this surprising discovery, it can be argued that the browning of an in vitro plant cell culture as a first response to elicitation would generally be interpreted as the result of phenoloxidation subsequent to cell damage. However, it has been found by Eilert and Constabel that cells exposed to elicitors over 24 to 48 hours do not suffer structural changes so that browning should be interpreted as due to oxidation of phenolics released from intact cells. Exposure of cells to elicitors over 72 hours does lead to some cell damage, and exposure of cells to elicitors over 96 hours results in general cell death. The observed tendency of elicited cells to produce brown cultures and to eventually undergo necrosis would thus make any reuse of the cells seem improbable, and contrary to the findings of the present invention. Moreover it has further been found that the elicited phytochemicals appear in the medium in quantities which allow for (a) extraction of the phytochemicals from the medium rather than the cells, and, (b) repeated elicitor treatments of such intact plant cells with consequent redution in the time needed to produce comparable quantities of such phytochemicals. These findings have allowed new, simple batch and semicontinuous methods for the production of such phytochemicals to be developed.

In accordance with the present invention it has been found that the timed medium replenishment and re-harvesting of elicited product, and repetitive elicitation-harvesting-replenishment-harvesting, using the same intact cells, is effective in the semi-continuous production of phytochemicals. For best results, re-elicitation requires recovery of elicited cells in fresh growth medium without elicitor sufficient to recondition the cells (e.g. at least 3 exchanges of medium at intervals of approximately 48 hours before second or subsequent elicitor additions).

Broadly speaking, therefore, this invention provides a semicontinuous method of producing phytochemicals using plant cells cultured in a cell growth medium with said plant cells being selected to produce phytochemicals when stimulated by a selected elicitor, comprising the steps of:

(a)  introducing a selected elicitor into the medium containing the plant cells thereby inducing the cells contained therein to produce phytochemicals;

(b)  recovering the phytochemicals produced by step (a) and intact cells;

(c)  replenishing said medium and removing any excess cells, and

(d)  after phytochemical accumulation, again recovering phytochemicals.

Steps (c) and (d) can be repeated on intact cells until the elicitor effect has worn off.  Subsequent elicitor additions may be made to intact cells.  Preferably steps (a) to (d) are followed by:

(e)  repeating step (c) sufficiently to recondition said intact cells, and then repeating steps (a) and (b), most preferably repeating steps (a) to (d).

The plant cells employed are preferably seed plant cells which are preferably cells from medicinal and industrially important plants.  Suitable plants include Papaver somniferum, Catharanthus roseus, Ruta graveolens, and others listed in Table II of "Microbial Technology", 2nd Edition, 1979, Vol. 1, Academic Press, page 409, and Table I of "Applied & Fundamental Aspects of Plant Cell Tissue & Organ Culture", Ed: J. Reinert & Y.P.S. Bajaj, Springer-Verlag, Ch. 6, p. 668-693, 1977, D.N. Butcher.  Cells from Lithospermum erythrorhizon (yielding shikonin) and from Coptis japonica (yielding berberin) are also suitable.

0226354

The method may be practised on plant cells contained in a cell suspension, a callus or an immobilized cell system.

Any suitable elicitor known in the art can be used. For example B. Wolters and U. Eilert in Dtsch. Apoth. Zeitg 123, 659-667 (1983) list just a few of the vast array of chemicals and physical conditions which can act as elicitors.

Because elicitors are most effective within a selected concentration range, the concentration of elicitor used should be chosen within a range to induce the production of the selected phytochemical.

The process of the invention is illustrated by the accompanying drawings, in which:

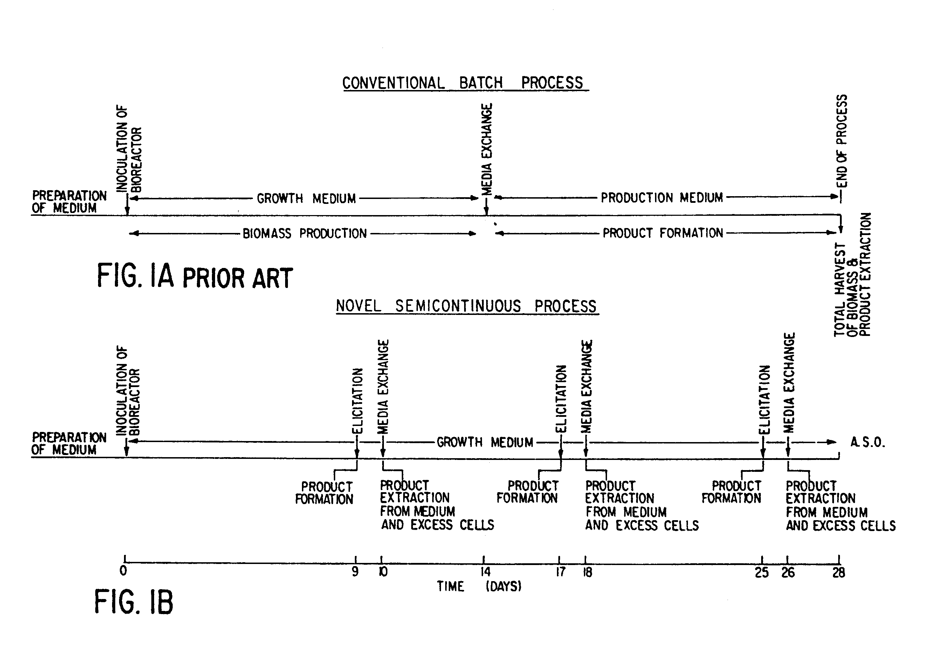

Figure 1A shows the steps of a conventional batch process and is to be compared with Figure 1B which shows the steps of one example of a novel semi-continuous process according to this invention.

Figures 2A, 2B and 2C show the steps and time frame of the three specific embodiments of the invention illustrated by the Examples presented hereinafter.

As already indicated, the process of this invention involves the repeated exposure of the plant cell cultures to biotic and/or abiotic elicitors and results in product formation within hours (for example, 12 to 24 hours for indole alkaloid accumulation in Catharanthus roseus cells). It does not require transfer of plant cells to a special production medium as synthesis of phytochemicals can be obtained as a result of elicitation in the growth medium. A further advantage of this method is to be found in the fact that much of the phytochemicals are released by the cells into the surrounding medium from where they can be extracted by conventional methods. Therefore, a total harvest of cells for extraction purposes as is now common practice, is no longer necessary, thus effectively shortening the time required for cell growth and metabolite production. As cell viability is not affected by elicitor treatment if the medium is replaced within about 24-72 hrs, depending on the cells, the cycle of cell reconditioning - product elicitation - product synthesis - product recovery can be repeated in a semicontinuous process so long as no other factors require the shutdown of the process. Initially the first elicitor treatment can be succssfully performed after the culture reaches sufficient cell density (usually 7-10 days). The product yield equals the amount obtained using conventional methods, however the time required to do so can be cut by up to two thirds.

The discovery that there is no need to transfer the cells from a growth medium to a production medium greatly simplifies the process of the production of phytochemicals from such plant cells.

Elicitation appears to be a transient reaction of the cells of a seed plant to stress, particularly the stress caused by the contact of the seed plant cells by foreign agents. As such, it is unforeseen that the phenomenon can be repeated on the same cells with similar, repeated responses in the production of phytochemicals. The repeatable response is exploited for the development of the semicontinuous method for the production of such phytochemicals.

The examples given illustrate that the method has wide application among different types of cells, and the amount and type of elicitors required vary. The amount of elicitor required to induce the production of a selected phytochemical depends upon the purity of the elicitor used (a homogenate or a defined chemical) and the sensitivity of the plant cells to the elicitor of choice. Generally, however, in an experimental procedure to determine the amount of elicitor giving the optimum response, the experimentor would work within the range of 0.1% to 5% v/v towards an optimum amount of elicitor. Also the times required for the different steps very and for any other cell-elicitor-phytochemical combination, these times would have to be determined by experimentation and optimization. How often elicitation is required, how often fresh medium has to be added and how often medium and phytochemicals can be removed is also a matter of experimentation with the particular cell-elicitor-phytochemical system of choice. How often the cells can be so treated before requiring a recovery period is also a matter of experimentation and optimization.

In the protocol of the examples given the repeated short-term exchange of the medium for product recovery might be replaced by continuous product extraction.

For general techniques in the area of plant tissue culture methods the user is referred to "Plant Tissue Culture Methods" edited by L.R. Wetter and F. Constabel, 2nd Revised Edition, National Research Council of Canada (1982).

In the examples given below the method of preparation of the elicitor homogenates was as described in U. Eilert et al, J. Plant. Physiol., 119, 65-76 (1985). This reference also gives details of the extraction of phytochemicals and other suitable elicitor/plant cell/product systems. The cell homogenization step was only employed when excess cells were harvested.

In some cases (e.g. in two of the examples) replacement of medium need not be accompanied every time by the addition of more elicitor, i.e. the elicited cells have a memory or hysteresis effect and continue to produce the phytochemicals in replenished media.

The following Examples are illustrative.

Example 1  The production of benzylisoquinoline alkaloids using Papaver somniferum cell suspension culture

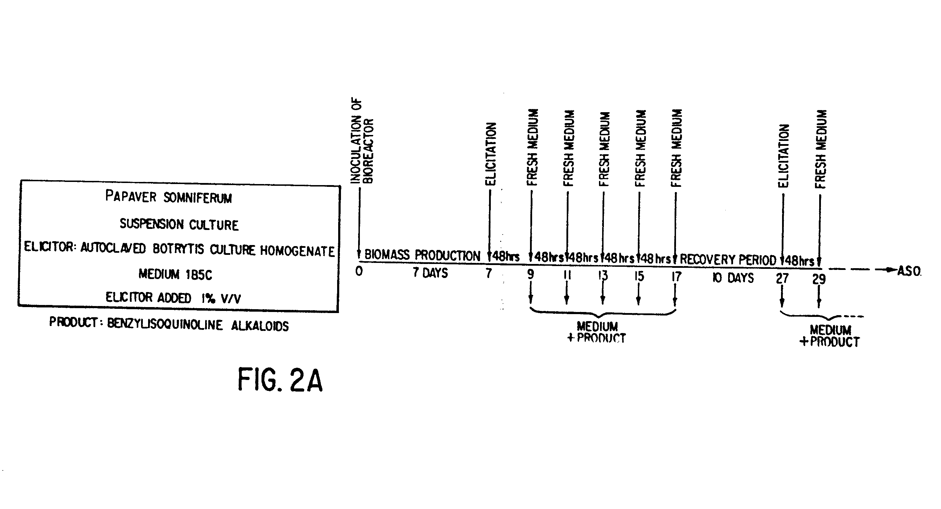

The schematic protocol for this example is shown in Figure 2A. 1B5C medium was inoculated with a suspension

- 8 -

of <u>Papaver</u> <u>somniferum</u> cells and grown for 7 days at the end of which time the cells were elicited with 1% v/v of auto-claved <u>Botrytis</u> culture homogenate. After a further 48 hrs the medium containing the benzylisoquinoline alkaloids was withdrawn and fresh growth medium added. This procedure of withdrawing medium and product and adding fresh growth medium as can be repeated up to four more times at 48 hr intervals before the cells require a recovery period of about 10 days before a further elicitation, withdrawal of medium and product, charging with fresh medium cycle can be performed.

Further tests with this system were carried out as follows.

### Fungal Elicitor Preparation

<u>Botrytis</u> spec. (PRL #2042) was taken from the fungal culture collection of Plant Biotechnology Institute/NRC, Saskatoon. One cm$^2$ pieces of mycelium which had been grown on B5-agar-medium without 2,4-D were used to inoculate 100 ml liquid B5-medium. These cultures were kept on a gyratory shaker (150 rpm) at room temperature in continuous low light for 6 days. The mycelium in its medium (100 ml) was homogenized and autoclaved for 20 min (121°C) yielding the elicitor preparation.

### Elicitation Experiments

For induction of alkaloid formation 1 ml elicitor was added to 100 ml of 5-day-old <u>Papaver</u> <u>somniferum</u> L.cv. Marianne cultures. 48 hrs later the culture medium was removed under sterile conditions and its alkaloid profile and content determined. Fresh weight of the wet cells was determined and the cells were then resuspended in fresh 1B5C-medium and the cultures adjusted to 100 ml for further subculturing. Several (3-7) additional media exchanges were performed at 48 hr intervals in the same way. After 10 media exchanges over 20 days, the cultures were diluted to original cell density (15-20 gFW/100 ml), subcultured for 2, 5 or 10 days and the cycle of elicitor treatment followed by media exchanges was repeated.

## Alkaloid extraction and analysis

For alkaloid extraction from the medium 5ml were brought to pH 9 with 0.1 N NaOH. Seven ml ethylacetate were added and homogenized for 2 min. After phase separation the ethylacetate layer was removed and the extraction repeated. The combined ethylacetate fractions were concentrated under nitrogen and used for qualitative and quantitative alkaloid analysis. The alkaloid pattern was determined by TLC on silica gel (Baker Si 250F) in the solvent systems (I) ethylacetate/MeOH (90:10); (II) ethylacetate. Alkaloids were detected using UV-light of 366 nm and 254 mm as well as daylight and Dragendorff's spraying reagent.

P. somniferum cell suspensions were cultured in the presence of Botrytis-homogenate for 7 days. Daily microscopic examination showed that most cells survived the first 48-96 hrs despite browning, but then cell deterioration became noticeable and after 5-7 days hardly any living cells were found. Three consecutive medium exchanges at 48 hr intervals after initial elicitation allowed survival of cells. Now subculturing of elicitor-treated cells was successful. Alkaloid profiles of the media were analysed and monitored. Table I gives the results of one representative experiment. Sanguinarine was the main alkaloid in the medium 48 and 96 hrs after addition of elicitor. After 96 hrs, in the second medium, i.e. the first replacement medium, the compound dihydrosanguinarine was noted. After 6 d, in the 3rd medium, i.e. 2nd replacement medium, this compound became the dominant alkaloid. The change in profile with dihydrosanguinarine becoming the main alkaloid occurred not only in the media, but also in the cells. No other alkaloids were detected in cells or media.

Response of cultures to repeated elicitor-treatment

After dilution of cells and standard subculturing for two 10 d periods in fresh 1-B5C medium, the alkaloid content of the first time elicitor-treated cells had become relatively low. Addition of elicitor to these cells caused a weaker and slower browning response of cells and medium than observed with cultures which had not been elicited before. Again, media were exchanged every 48 hr after elicitation and monitored for alkaloid content (Table II). Whereas dihydrosanguinarine was the main compound before re-elicitation, 48 hrs after re-elicitation sanguinarine level had increased and surpassed the level of dihydrosanguinarine.

After another 48-96 hrs the alkaloid profile gradually shifted back to dihydrosanguinarine formation (Table II).

Table I  Alkaloid content of culture media of <u>Papaver somniferum</u> cells 2 d after first elicitation and 2 d after the 1st and 2nd exchange of culture medium.

| Interval After Elicitation (d) | Medium | Sanguinarine µg/100 ml Culture | Dihydrosan-guinarine µg/100 ml |
|---|---|---|---|
| 0 - 2 | Medium with elicitor | 363 | 58 |
| 2 - 4 | 1st exchange medium | 17519 | 1108 |
| 4 - 6 | 2nd exchange medium | 4206 | 7442 |
| Control (non-elicited culture) 0 - 6 | | – | – |

Cell line: #2009, 20 g.FW/100 ml culture, 0.75% Botrytis elicitor.
\- = no alkaloid detected.

B5 and B5C media are described in Gamborg et al, Exp. Cell Res. 50: p.151-158, 1968.

Abbreviations:

    FW = Cell Fresh Weight

  Sge = sanguinarine

 Dsge = dihydrosanguinarine

Table II　Alkaloid Content of culture media from
re-elicited cultures, replaced in 48 hr
intervals after fresh elicitor treatment.

| Interval (days) | Sanguinarine $\mu$g/100 ml | Dihydrosanguinarine $\mu$g/100 ml | Ratio Sge:Dsge |
|---|---|---|---|
| Control medium | -- | 293 | -- |
| 0-2 | 300 | 206 | 1:0.7 |
| 2-4 | 131 | 1206 | 1:9 |
| 4-6 | trace | 172 | -- |
| 6-8 | 10 | 411 | 1:41 |
| 8-10 | 31 | 3502 | 1:112 |

Cell line #2009 re-elicited; 15 g FW/100 ml culture;
0.75% Botrytis elicitor; alkaloid content of control
cells; Sge. 2.5 $\mu$g/g FW, Dsge: 21 $\mu$g/g FW.

## Semicontinuous production of benzophenanthridines

Table III shows the results of one representative
long-term experiment to produce benzophenanthridines semi-
continuously by repeated elicitation.　A one-week-old subculture of cells, which had undergone elicitation and one
re-elicitation cycle, was again treated with elicitor, and
the medium exchanged every 48 hr.　Over a 12 day period cells
excreted between 15 and 50 $\mu$g alkaloid per g cell fresh weight
into 75 ml medium on a daily basis.　Alkaloid levels were
highest in the 2nd and 3rd medium.　After the 6th medium
exchange, 12 days after re-elicitation, cells were subcultured
for 5 days, and then treated with fresh elicitor.　The culture
responded with increased benzophenanthridine excretion;

TABLE III

Semi-continuous alkaloid production by re-elicitation of P. somniferum cells and regular replacement of culture media. (1-week old subculture)

| Interval (days) | Sanguinarine µg/100 ml | Dihydro- sanguinarine µg/100 ml | Ratio Sge: Dsge | Cell Density FW g/100 ml |
|---|---|---|---|---|
| Control | - | - | - | 14 |
| 2nd re-elicitation | | | | |
| 0-2 | 82 | 5 | 1:0.06 | 14 |
| 2-4 | 244 | 4 | 1:0.02 | 15 |
| 4-6 | 64 | 1644 | 1:26 | 16 |
| 6-8 | 52 | 754 | 1:14.5 | 18 |
| 8-10 | 73 | 760 | 1:10.5 | 18 |
| 10-12 | 35 | 784 | 1:22 | 19 |
| 12-17 | 8 | 753 | 1:94 | 40 |
| 3rd re-elicitation | | | | |
| 17-19 | 495 | 3486 | 1:7 | 25 |
| 19-21 | 222 | 7704 | 1:34 | 28 |
| 21-23 | 159 | 4536 | 1:29 | 30 |
| 23-25 | 72 | 3454 | 1:48 | 31 |
| 25-27 | 48 | 2408 | 1:50 | 34 |
| 27-29 | 78 | 2766 | 1:35 | 36 |
| 29-31 | 156 | 3225 | 1:21 | 37 |
| 4th re-elicitation | | | | |
| 31-33 | 2432 | 2774 | 1:1.1 | 21 |
| 33-35 | 2582 | 1495 | 1:0.6 | 22 |
| 35-37 | 1150 | 1705 | 1:1.5 | 23 |

dihydrosanguinarine was the main alkaloid. After re-elicitation the transient switch to a higher sanguinarine formation was observed again. As production declined with time cells were diluted to initial cell density and re-elicited. In response benzophenanthridine accumulation in the medium increased 10-fold but declined during the next intervals somewhat more rapidly than observed before.

#### Example 2 The Production of Indole Alkaloids Using Catharanthus roseus Suspension Culture

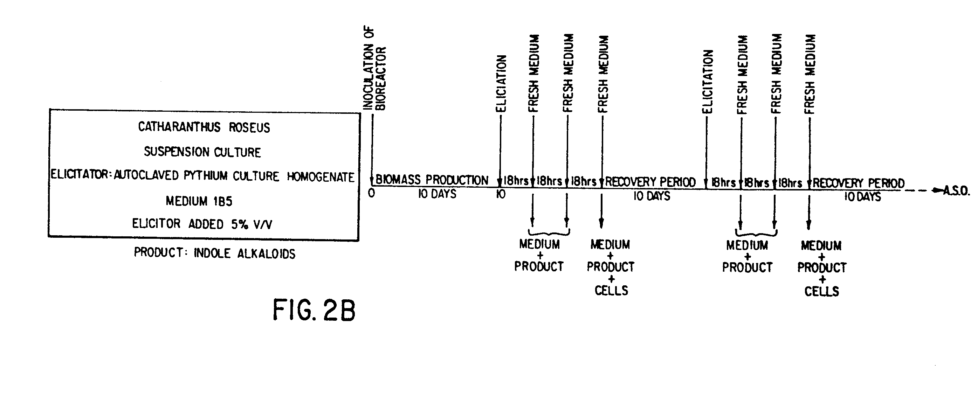

The schematic protocol for this example is shown in Figure 2B. 1B5 medium was inoculated with a suspension of Catharanthus roseus cells and grown for 10 days at the end of which time the cells were elicited with 5% v/v autoclaved Pythium culture homogenate. After a further 18 hrs the medium containing indole alkaloids was withdrawn and fresh growth medium added. This procedure of withdrawing medium and product and adding fresh growth medium can be repeated two more times (excess cells were alos withdrawn on the last occasion) every 18 hrs before the cells require a recovery period of about 10 d before another cycle of elicitation, withdrawal of medium and product, charging with fresh medium, can be performed.

In further tests, upon treatment of 5 cell lines of Catharanthus roseus with homogenates of various fungi, as well as with chemically defined phytoalexin elicitors, all except one (non-alkaloid producing #916) responded with browning and accumulation of tryptamine within 6-24 hr. Cells of line #615 responded with not only accumulating tryptamine, but also N-acetyl tryptamine, strictosidine lactam, ajmalicine, tabersonine, lochnericine, and catharanthine. Based on amounts of alkaloids accumulated, cells of line #615 performed best when treated with elicitor homogenates of <u>Alternaria zinnae</u>, <u>Pythium aphanidermatum</u>, <u>Verticillium dahliae</u>, and <u>Rhodotorula rubra</u>. A Phythium homogenate concentration of 5% and a Rhodotorula homogenate concentration of 0.5% effected maximum alkaloid yields and, thus, were used in subsequent studies. These revealed a temporary increase of the level of alkaloids in cells and in their medium after 12-24 hr of treatment. Tenday old subcultures responded better than younger and older ones. The elicitor stimulated accumulation of alkaloids and alkaloid composition did not depend on the use of 1-B5 or alkaloid production medium. A 5 L cell suspension of #615 grown in a 7.5 L bioreactor and treated with 5% Pythium homogenate for 18 hr was found to contain strictosidine lactam, ajmalicine and catharanthine in concentrations of 27, 10 and 13 $\mu$g/g DW respectively, the medium contained 42% of total ajmalicine.

<u>Example 3    The Production of Acridone Alkaloids Using</u>
<u>Ruta graveolens Cells Suspension Culture</u>

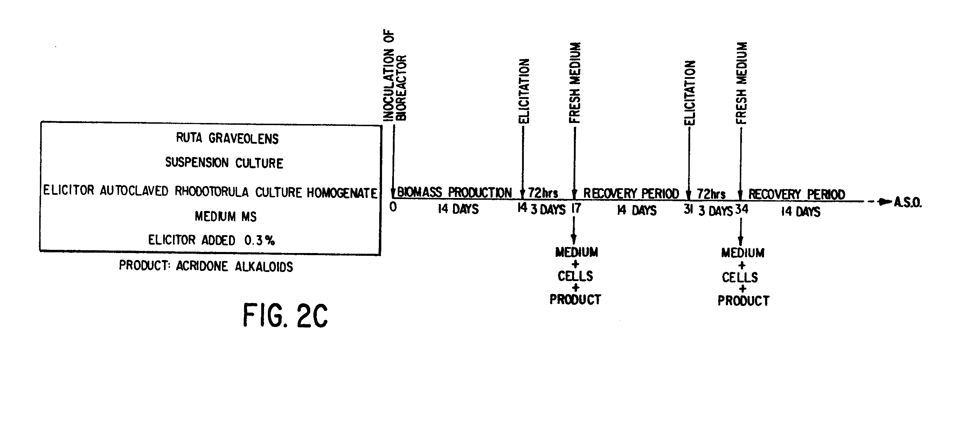

The schematic protocol for this example is shown in Figure 2C. MS medium was inoculated with a suspension of <u>Ruta graveolens</u> cells and grown for 14 days at the end of which time the cells were elicited with 0.3% v/v of auto-claved <u>Rhodotorula</u> culture homogenate.  After a further 3 days the medium containing excess cells and acridone alkaloids were withdrawn and fresh growth medium added.  The cells are allowed to recover for about 14 days before a further elicitation, withdrawal of medium, cells and product, charging with fresh medium cycle was performed.

## CLAIMS

1.    A method for the production of a . given phytochemical, which comprises stimulating an <u>in vitro</u> culture medium containing plant cells capable of producing said phytochemical with an elicitor capable of stimulating the production of the phytochemical in said medium, and recovering the accumulated phytochemical from said medium, characterised in that the method is carried out on a semi-continuous basis by:

(a)    introducing the elicitor into the culture medium containing the plant cells, thereby to induce the cells contained therein to produce the desired phytochemical;

(b)    separating from said culture medium a first quantity of medium containing intact cells and product phytochemical;

(c)    replenishing the remaining medium and optionally adding add tional elicitor;

(d)    separating a second quantity of medium containing excess cells and a further quantity of product phytochemical;

(e)    recovering  the product phytochemical from the first and second quantities of medium separated in steps (b) and (d); and optionally repeating steps (b) to (e) on a cyclical basis.

2.    A method according to claim 1, characterised in that steps (c) and (d) are repeated at least once.

3.    A method according to claim 1 or 2, characterised in that the culture medium is re-elicited at least once by the addition of fresh elicitor to the medium after separation of the medium containing excess cells and product phytochemicals.

4.    A method according to claim 3, characterised in that the re-elicitation takes place after the replenishment of said medium and after allowing the replenished medium to undergo a period of recovery.

5.    A method according to claim 3 or 4, characterised in that the re-elicitation and recovery of phytochemicals is repeated on the same cells until the yield of phytochemical becomes insufficient.

6.      A method according to any one of claims 1-4, characterised in that the plant cells are seed plant cells.

7.      A method according to any one of claims 1-6, characterised in that the cells used are contained in a cell suspension, a callus or an immobilized cell system.

8.      A method according to any one of claims 1-7, characterised in that the elicitor is utilized in step (a) at a concentration that induces the optimum production of the desired phytochemical.

9.      A method according to claim 8, characterised in that the concentration of elicitor in the medium is from 0.1% to 5% v/v.

10.      A method according to any one of claims 1-8, characterised in that the plant cells are cells of Papaver somniferum, Catharanthus roseus or Ruta graveolens.

11.      A method according to claim 10, characterised in that the plant cells are Papaver somniferum cells, the elicitor is autoclaved Botrytis culture homogenate and the concentration of elicitor is about 1% v/v.

12.      A method according to claim 10, characterised in that the plant cells are Catharanthus roseus cells, the elicitor is an autoclaved Pythium culture homogenate and the concentration of elicitor is about 5% v/v.

13.      A method according to claim 10, characterised in that the plant cells are Ruta graveolens cells, the elicitor is autoclaved Rhodotorula culture homogenate and the concentration of elicitor is about 0.3% v/v.

# FIG. IA PRIOR ART

## CONVENTIONAL BATCH PROCESS

PREPARATION OF MEDIUM

INOCULATION OF BIOREACTOR

GROWTH MEDIUM

MEDIA EXCHANGE

PRODUCTION MEDIUM

PRODUCT FORMATION

END OF PROCESS

BIOMASS PRODUCTION

PRODUCT FORMATION

TOTAL HARVEST OF BIOMASS & PRODUCT EXTRACTION

# FIG. IB

## NOVEL SEMICONTINUOUS PROCESS

PREPARATION OF MEDIUM

INOCULATION OF BIOREACTOR

PRODUCT FORMATION — ELICITATION

PRODUCT EXTRACTION FROM MEDIUM AND EXCESS CELLS — MEDIA EXCHANGE

GROWTH MEDIUM

PRODUCT FORMATION — ELICITATION

PRODUCT EXTRACTION FROM MEDIUM AND EXCESS CELLS — MEDIA EXCHANGE

PRODUCT FORMATION — ELICITATION

PRODUCT EXTRACTION FROM MEDIUM AND EXCESS CELLS — MEDIA EXCHANGE

A.S.O.

TIME (DAYS)

0    9    10    14    17    18    25    26    28

FIG. 2A

PAPAVER SOMNIFERUM

SUSPENSION CULTURE

ELICITOR: AUTOCLAVED BOTRYTIS CULTURE HOMOGENATE

MEDIUM 1B5C

ELICITOR ADDED 1% V/V

PRODUCT: BENZYLISOQUINOLINE ALKALOIDS

FIG. 2B

CATHARANTHUS ROSEUS

SUSPENSION CULTURE

ELICITATOR: AUTOCLAVED PYTHIUM CULTURE HOMOGENATE

MEDIUM 1B5

ELICITOR ADDED 5% V/V

PRODUCT: INDOLE ALKALOIDS

INOCULATION OF BIOREACTOR

ELICIATION

FRESH MEDIUM

FRESH MEDIUM

FRESH MEDIUM

ELICITATION

FRESH MEDIUM

FRESH MEDIUM

FRESH MEDIUM

BIOMASS PRODUCTION | 18hrs | 18hrs | 18hrs | RECOVERY PERIOD | 18hrs | 18hrs | 18hrs | RECOVERY PERIOD — — — A.S.O.

0            10 DAYS            10                    10 DAYS                              10 DAYS

MEDIUM + PRODUCT

MEDIUM + PRODUCT + CELLS

MEDIUM + PRODUCT

MEDIUM + PRODUCT + CELLS

FIG. 2C

RUTA GRAVEOLENS

SUSPENSION CULTURE

ELICITOR AUTOCLAVED RHODOTORULA CULTURE HOMOGENATE

MEDIUM MS

ELICITOR ADDED 0.3%

PRODUCT: ACRIDONE ALKALOIDS

INOCULATION OF BIOREACTOR

ELICITATION

FRESH MEDIUM

ELICITATION

FRESH MEDIUM

BIOMASS PRODUCTION    72hrs    RECOVERY PERIOD    72hrs    RECOVERY PERIOD    — ► A.S.O.

0        14 DAYS        14 3 DAYS 17      14 DAYS      31 3 DAYS 34      14 DAYS

MEDIUM
+
CELLS
+
PRODUCT

MEDIUM
+
CELLS
+
PRODUCT

4/4

0226354